# EUROPÄISCHE PATENTSCHRIFT

(11) **EP 0 929 513 B1**
(45) Veröffentlichungstag und Bekanntmachung des Hinweises auf die Patenterteilung: **11.04.2001**
(21) Anmeldenummer: 97941948.8
(22) Anmeldetag: 21.08.1997
(51) Int. Cl.: C07C 209/48, C07C 253/30

(54) **VERFAHREN ZUR GLEICHZEITIGEN HERSTELLUNG VON 6-AMINOCAPRONITRIL UND HEXAMETHYLENDIAMIN**
PROCESS FOR SIMULTANEOUSLY PREPARING 6-AMINOCAPRONITRILE AND HEXAMETHYLENE DIAMINE
PROCEDE DE PREPARATION SIMULTANEE DE 6-AMINOCAPRONITRILE ET DE DIAMINE D'HEXAMETHYLENE

(30) Priorität: 10.09.1996 DE 19636765
(43) Veröffentlichungstag der Anmeldung: 21.07.1999
(73) Patentinhaber: BASF AKTIENGESELLSCHAFT, 67056 Ludwigshafen (DE)
(72) Erfinder: VOIT, Guido, D-69198 Schriesheim (DE); FLICK, Klemens, D-76863 Herxheim (DE); LUYKEN, Hermann, D-67069 Ludwigshafen (DE); REHFINGER, Alwin, D-67112 Mutterstadt (DE); BASSLER, Peter, D-68519 Viernheim (DE)
(86) Internationale Anmeldenummer: EP9704542
(87) Internationale Veröffentlichungsnummer: WO9811051

(56) Entgegenhaltungen:
- EP-A- 0 077 911
- WO-A-93/12073
- WO-A-96/23802
- DE-A- 4 235 466
- DE-A- 19 500 222
- DE-A- 19 548 289
- GB-A- 2 212 155

## Beschreibung

Die vorliegende Erfindung betrifft ein Verfahren zur gleichzeitigen Herstellung von 6-Aminocapronitril und Hexamethylendiamin ausgehend von einem Gemisch, das eine phosphorhaltige Verbindung und im wesentlichen Adipodinitril enthält und das durch Umsetzung von Butadien oder Pentennitrilen mit Cyanwasserstoffsäure in Gegenwart eines eine phosphorhaltige Verbindung enthaltenden Katalysators erhalten wurde.

Die Herstellung von Adipodinitril durch Umsetzung von Butadien oder Pentennitrilen mit Cyanwasserstoffsäure in Gegenwart eines eine phosphorhaltige Verbindung enthaltenden Katalysators ist allgemein bekannt, beispielsweise aus: K.Weissermel, H.-J.Arpe, Industrielle Organische Chemie, 3. Auflage, VCH Verlagsgesellschaft mbH, Weinheim, 1988, Seite 263-264, Verfahren 3.

Aus der DE-A 19 500 222 und der Deutschen Anmeldung 19 548 289.1 ist ein Verfahren zur gleichzeitigen Herstellung von 6-Amincapronitril und Hexamethylendiamin durch partielle Hydrierung von Adipodinitril in Gegenwart eines Katalysators, die Abtrennung von Hexamethylendiamin und 6-Aminocapronitril aus der Mischung und Umsetzung von 6-Aminocapronitril zu Caprolactam sowie Rückführung eines im wesentlichen aus Adipodinitril bestehenden Teils des Rückstands in das Verfahren bekannt.

Nachteilig bei dem Einsatz von Adipodinitril, das durch Umsetzung von Butadien oder Pentennitrilen mit Cyanwasserstoffsäure in Gegenwart eines eine phosphorhaltige Verbindung enthaltenden Katalysators erhalten wurde, bei der genannten partiellen Hydrierung ist, das der für die Hydrierung eingesetzte Katalysator eine rasche Desaktivierung und schlechte 6-Aminocapronitril-Ausbeuten und -Selektivitäten zeigt.

Aufgabe der vorliegenden Erfindung war es daher, ein Verfahren zur gleichzeitigen Herstellung von 6-Aminocapronitril und Hexamethylendiamin ausgehend von einem Gemisch, das eine phosphorhaltige Verbindung und im wesentlichen Adipodinitril enthält und das durch Umsetzung von Butadien mit Cyanwasserstoffsäure in Gegenwart eines eine phosphorhaltige Verbindung enthaltenden Katalysators erhalten wurde, zur Verfügung zu stellen, das die genannten Nachteile nicht aufweist, und die gleichzeitige Herstellung von 6-Aminocapronitril und Hexamethylendiamin ausgehend von einem Gemisch, das eine phosphorhaltige Verbindung und im wesentlichen Adipodinitril enthält und das durch Umsetzung von Butadien mit Cyanwasserstoffsäure in Gegenwart eines eine phosphorhaltige Verbindung enthaltenden Katalysators erhalten wurde, auf technisch einfache und wirtschaftliche Weise ermöglicht.

Demgemäß wurde ein Verfahren zur gleichzeitigen Herstellung von 6-Aminocapronitril und Hexamethylendiamin ausgehend von einem Gemisch, das eine phosphorhaltige Verbindung und im wesentlichen Adipodinitril enthält und das durch Umsetzung von Butadien mit Cyanwasserstoffsäure in Gegenwart eines eine phosphorhaltige Verbindung enthaltenden Katalysators erhalten wurde, dadurch gekennzeichnet, daß man
(1) den Gewichtsanteil an einer phosphorhaltigen Verbindung, gerechnet als Phosphor, in dem Gemisch reduziert und
(2) die erhaltene Mischung in Gegenwart eines Katalysators zu einem alpha, omega-Aminonitril partiell hydriert und
(3) 6-Aminocapronitril und Hexamethylendiamin aus der Mischung abtrennt,
gefunden.

Die Herstellung von Adipodinitril aus Butadien und Cyanwasserstoffsäure kann man nach einem der bekannten Verfahren durchführen, beispielsweise nach einem der zuvor genannten Verfahren beschrieben in: K.Weissermel, H.-J.Arpe, Industrielle Organische Chemie, 3. Auflage, VCH Verlagsgesellschaft mbH, Weinheim, 1988, Seite 263-264, Verfahren 3, indem man im allgemeinen die Umsetzung vorzugsweise in flüssiger Phase an Katalysatoren durchführt, die als aktive Komponente eine Komplexverbindung aus einem Metall wie Nickel mit phosphorhaltigen Liganden wie Phosphin- oder Phosphit-Verbindungen, beispielsweise [(C₆H₅)₃PO]₄Ni, enthalten. Im allgemeinen führt man die Umsetzung mehrstufig durch, wobei in der ersten Stufe ein Gemisch isomerer Pentennitrile und Methylbuthennitrile erhalten wird, das anschließend zu vorwiegend 3- und 4-Pentennitrilen isomerisiert wird. In einer zweiten Stufe wird durch erneute Anlagerung von Cyanwasserstoffsäure in Gegenwart der genannten Katalysatoren Adipodinitril gebildet.

Das nach diesem Verfahren hergestellte Adipodinitril enthält mindestens eine phosphorhaltige Verbindung, im allgemeinen in Gewichtsanteilen von 1 bis 50 ppm gerechnet als Phosphor, bezogen auf Adipodinitril. Im folgenden wird eine phosphorhaltige Verbindung, wie auch, im Falle mehrerer Verbindungen, die Summe der phosphorhaltigen Verbindungen gleichermaßen als eine phosphorhaltige Verbindung bezeichnet.

Der Gewichtsanteil an der phosphorhaltigen Verbindung wird anschließend erfindungsgemäß reduziert, wodurch man vorteilhaft Gewichtsanteile an phosphorhaltiger Verbindung, gerechnet als Phosphor, bezogen auf Adipodinitril, von kleiner 5 ppm, vorzugsweise kleiner als 1 ppm erhält.

Zur Reduzierung des Gewichtsanteils an einer phosphorhaltigen Verbindung in dem Gemisch kommen verschiedene, an sich bekannte Verfahren, wie Fällung, vorzugsweise Extraktion, Behandlung mit einer Base wie Natronlauge oder Kalilauge, Adsorption oder Chemisorption, insbesondere an ein Metalloxiden wie Calciumoxid oder besonders bevorzugt Destillation in Betracht.

Die Destillation kann man dabei vorteilhaft bei Drücken von 1 bis 100 mbar, vorzugsweise 10 bis 200 mbar durchführen, wobei das Adipodinitril meist als Kopfprodukt anfällt, da die phosphorhaltige Verbindung im wesentlichen schwerflüchtiger als Adipodinitril ist.

Die partielle Hydrierung von Adipodinitril kann man nach einem der bekannten Verfahren durchführen, beispielsweise nach einem der zuvor genannten Verfahren beschrieben in US 4 601 8591, US 2 762 835, US 2 208 598, DE-A 848 654, DE-A 954 4161 DE-A 4 235 466 oder WO 92/21650, indem man im allgemeinen die Hydrierung in Gegenwart von Nickel-, Cobalt-, Eisen- oder Rhodium-haltigen Katalysatoren durchführt. Dabei können die Katalysatoren als. Trägerkatalysatoren oder als Vollkatalysatoren verwendet werden. Als Katalysatorträger kommen beispielsweise Aluminiumoxid, Siliciumdioxid, Titandioxid, Magnesiumoxid₁ Aktivkohlen und Spinelle in Frage. Als Vollkatalysatoren kommen beispielsweise Raney-Nickel und Raney-Cobalt in Betracht.

Üblicherweise wählt man die Katalysatorbelastung im Bereich von 0,05 bis 10, vorzugsweise von 0,1 bis 5 kg Adipodinitril/l_{Katalysator}*h.

Die Hydrierung nimmt man in der Regel bei Temperaturen im Bereich von 20 bis 200, vorzugsweise von 50 bis 150°C, und bei Wasserstoff-Partialdrücken von 0,1 bis 40, vorzugsweise von 0,5 bis 30 MPa, vor.

Bevorzugt führt man die Hydrierung in Gegenwart eines Lösungsmittels, insbesondere Ammoniak, durch. Die Ammoniak-Menge wählt man im allgemeinen im Bereich von 0,1 bis 10, vorzugsweise von 0,5 bis 3 kg Ammoniak/kg Adipodinitril.

Das Molverhältnis von 6-Aminocapronitril zu Hexamethylendiamin, und damit das Molverhältnis von Caprolactam zu Hexamethylendiamin, kann durch den jeweils gewählten Adipodinitril-Umsatz gesteuert werden. Bevorzugt arbeitet man bei Adipodinitril-Umsätzen im Bereich von 10 bis 90, bevorzugt von 30 bis 80%, um hohe 6-Aminocapronitril-Selektivitäten zu erhalten.

In der Regel liegt die Summe aus 6-Aminocapronitril und Hexamethylendiamin je nach Katalysator und Reaktionsbedingungen bei 95 bis 99%, wobei als mengenmäßig bedeutendstes Nebenprodukt Hexamethylenimin auftritt.

In einer bevorzugten Ausführungsform nimmt man die Umsetzung in Gegenwart von Ammoniak und Lithiumhydroxid, oder einer Lithiumverbindung, die unter den Reaktionsbedingungen Lithiumhydroxid bildet, bei Temperaturen im Bereich von 40 bis 120, vorzugsweise von 50 bis 100, besonders vorzugsweise von 60 bis 90°C vor; den Druck wählt man im allgemeinen im Bereich von 2 bis 12, vorzugsweise von 3 bis 10, besonders bevorzugt von 4 bis 8 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bevorzugt wählt man die Druck- und Temperaturbereiche so, daß man die Umsetzung in flüssiger Phase vornehmen kann.

Ammoniak setzt man im allgemeinen in einer Menge ein, so daß das Gewichtsverhältnis von Ammoniak zu Dinitril im Bereich von 9:1 bis 0,1:1, bevorzugt von 2,3:1 bis 0,25:1, besonders bevorzugt von 1,5:1 bis 0,4:1, liegt.

Die Menge an Lithiumhydroxid wählt man in der Regel im Bereich von 0,1 bis 20, bevorzugt von 1 bis 10 Gew.-%, bezogen auf die Menge an eingesetztem Katalysator.

Als Lithiumverbindungen, die unter den Reaktionsbedingungen Lithiumhydroxid bilden, seien genannt: Lithiummetall, Alkyl- und Aryllithiumverbindungen wie n-Butyllithium und Phenyllithium. Die Menge an diesen Verbindungen wählt man im allgemeinen so, daß die zuvor genannte Menge an Lithiumhydroxid erhalten wird.

Als Katalysatoren setzt man bevorzugt Nickel-, Ruthenium-, Rhodium-, Eisen- und Cobalt-haltige Verbindungen ein, bevorzugt solche vom Raney-Typ, insbesondere Raney-Nickel und Raney-Cobalt. Man kann die Katalysatoren auch als Trägerkatalysatoren einsetzen, wobei als Träger beispielsweise Aluminiumoxid, Siliciumdioxid, Zinkoxid, Aktivkohle oder Titandioxid dienen können. (s. Appl. Het. Cat., 1987, S. 106-122; Catalysis, Vol. 4 (1981) S. 1-30). Besonders bevorzugt ist Raney-Nickel (beispielsweise von BASF AG, Degussa und Grace).

Die Nickel-, Ruthenium-, Rhodium-, Eisen- und Cobalt-Katalysatoren können mit Metallen der Gruppe VIB (Cr, Mo, W) und VIII (Fe, Ru, Os, Co (nur im Falle von Nickel), Rh, Ir, Pd, Pt) des Periodensystems modifiziert sein. Nach bisherigen Beobachtungen führt der Einsatz von insbesondere modifizierten Raney-Nickel-Katalysatoren, beispielsweise mit Chrom und/oder Eisen modifiziert, zu höheren Aminonitril-Selektivitäten. (Herstellung siehe DE-A 2 260 978; Bull. Soc. Chem. 13 (1946) S. 208).

Die Menge an Katalysator wählt man im allgemeinen so, daß die Cobalt-, Ruthenium-, Rhodium-, Eisen- oder Nickel-Menge im Bereich von 1 bis 50, bevorzugt von 5 bis 20 Gew.-%, bezogen auf die eingesetzte Menge an Dinitril, beträgt.

Die Katalysatoren können als Festbettkatalysatoren in Sumpf- oder Rieselfahrweise oder als Suspensionskatalysatoren eingesetzt werden.

In einer weiteren bevorzugten Ausführungsform hydriert man Adipodinitril partiell zu 6-Aminocapronitril bei erhöhter Temperatur und erhöhtem Druck in Gegenwart eines Lösungsmittels und eines Katalysators, indem man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a), eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a), einer Verbindung auf der Basis eines Alkalimetalles oder eines Erdalkalimetalles, enthält,
mit der Maßgabe, daß, wenn als Komponente (a) eine Verbindung auf der Basis von nur Ruthenium oder Rhodium oder Ruthenium und Rhodium oder Nickel und Rhodium gewählt wird, der Promotor (b) gewünschtenfalls entfallen kann, sowie mit der weiteren Maßgabe, daß, die Komponente (a) nicht auf der Basis von Eisen besteht, wenn die Komponente (b) Aluminium ist.

Bevorzugte Katalysatoren sind solche, in denen die Komponente (a) mindestens eine Verbindung auf der Basis eines Metalles, ausgewählt aus der Gruppe aus Nickel, Cobalt und Eisen, in einer Menge im Bereich von 10 bis 95 Gew.-% sowie Ruthenium und/oder Rhodium in einer Menge im Bereich von 0,1 bis 5 Gew.-%, jeweils bezogen auf die Summe der Komponenten (a) bis (c), enthält,

die Komponente (b) mindestens einen Promotor auf der Basis eines Metalles, ausgewählt aus der Gruppe bestehend aus Silber, Kupfer, Mangan, Rhenium, Blei und Phosphor, in einer Menge im Bereich von 0,1 bis 5 Gew.-%, bezogen auf (a), enthält, und

die Komponente (c) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von 0,1 bis 5 Gew.-% enthält.

Besonders bevorzugte Katalysatoren sind:
Katalysator A, enthaltend 90 Gew.-% Cobaltoxid (CoO), 5 Gew.-% Manganoxid (Mn₂O₃), 3 Gew.-% Phosphorpentoxid und 2 Gew.-% Natriumoxid (Na₂O),
Katalysator B, enthaltend 20 Gew.-% Cobaltoxid (CoO), 5 Gew.-% Manganoxid (Mn₂O₃), 0,3 Gew.-% Silberoxid (Ag₂O), 70 Gew.-% Siliciumdioxid (SiO₂), 3,5 Gew.-% Aluminiumoxid (Al₂O₃), 0,4 Gew.-% Eisenoxid (Fe₂O₃), 0,4 Gew.-% Magnesiumoxid (MgO) sowie 0,4 Gew.-% Calciumoxid (CaO), und
Katalysator C, enthaltend 20 Gew.-% Nickeloxid (NiO), 67,42 Gew.-% Siliciumdioxid (SiO₂), 3,7 Gew.-% Aluminiumoxid (Al₂O₃), 0,8 Gew.-% Eisenoxid (Fe₂O₃), 0,76 Gew.-% Magnesiumoxid (MgO),
1,92 Gew.-% Calciumoxid (CaO), 3,4 Gew.-% Natriumoxid (Na₂O) sowie 2,0 Gew.-% Kaliumoxid (K₂O).

Solche Katalysatoren sind beispielsweise in DE-A 195 002 22 und der Deutschen Anmeldung 195 482 89.1 beschrieben.

Besonders bevorzugte Katalysatoren sind solche, die
a) eine Verbindung auf der Basis von Eisen wie Eisenoxid enthalten und
b) von 0 bis 5 Gew.-% bezogen auf (a) eines Promotors auf der Basis eines Elementes oder 2,3,4 oder 5 Elementen ausgewählt aus der Gruppe bestehend aus Aluminium, Silizium, Zirkonium, Vanadium und Titan sowie
c) von 0 bis 5 Gew.-%, vorzugsweise 0,1 bis 3 Gew.-%, insbesondere 0,1 bis 0,5 Gew.-% bezogen auf (a) einer Verbindung auf der Basis eines Alkali- oder Erdalkalimetalles, vorzugsweise ausgewählt aus der Gruppe bestehend aus Lithium, Natrium, Kalium, Rubidium, Cäsium, Magnesium und Calcium.

Bei den bevorzugt einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponenten (a) zusammen mit Vorläufern der Promotoren (Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle wie Nitrate, Chloride, Acetate, Formiate und Sulfate sowie insbesondere Hexachloroplatinat in Betracht, vorzugsweise Nitrate und Hexachloroplatinat.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 80 bis 250, vorzugsweise von 80 bis 180°C bei Katalysatoren auf der Basis von Ruthenium oder Rhodium als Komponente (a), oder im Bereich von 200 bis 500, vorzugsweise von 250 bis 400°C bei Katalysatoren auf der Basis eines der Metalle ausgewählt aus der Gruppe aus Nickel, Cobalt und Eisen als Komponente (a) 2 bis 24 h einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 1 pro 1 Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die Katalysatoren können als Festbettkatalysatoren in Sumpf- oder Rieselfahrweise oder als Suspensionskatalysatoren eingesetzt werden.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 30, vorzugsweise von 3 bis 30, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 40, vorzugsweise von 3 bis 30 MPa wählt. Bevorzugt führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Die partielle Hydrierung kann man in einem üblichen hierfür geeigneten Reaktor (R1 in Zeichnung) durchführen.

Bei der Hydrierung erhält man eine Mischung, die 6-Aminocapronitril, Hexamethylendiamin und Adipodinitril enthält.

Die Abtrennung von 6-Aminocapronitril, Hexamethylendiamin und einem im wesentlichen Adiponitril enthaltenden Teil von der Mischung kann in an sich bekannter Weise, vorzugsweise destillativ, beispielsweise gemäß der DE-A 195 002 22 oder der Deutschen Anmeldung 19 548 289.1, gleichzeitig oder nacheinander erfolgen.

Die Destillation in der ersten Kolonne K1 in der Zeichnung) führt man dabei so durch, daß man die Mischung, enthaltend im wesentlichen 6-Aminocapronitril, Hexamethylendiamin, Ammoniak, Adipodinitril und Hexamethylenimin, bevorzugt eine Mischung, enthaltend im wesentlichen von 1 bis 70, vorzugsweise von 5 bis 40 Gew.-% 6-Aminocapronitril,
- von 1 bis 70,: vorzugsweise von 5 bis 40 Gew.-% Adipodinitril,
- von 0,1 bis 70,: vorzugsweise von 1 bis 40 Gew.-% Hexamethylendiamin,
- von 0,01 bis 10,: vorzugsweise von 0,05 bis 5 Gew.-% Hexamethylenimin und
- von 5 bis 95,: vorzugsweise von 20 bis 85 Gew.-% Ammoniak, in der Regel in einer üblichen Destillationskolonne, bei einer Sumpftemperatur im Bereich von 60 bis 250, vorzugsweise von 100 bis 200°C und einem Druck im Bereich von 5 bis 30, vorzugsweise von 12 bis 25 bar in Gegenwart von einer oder mehreren unter den Destillationsbedingungen inerten Verbindungen A durchführt, die bei einem Druck von 18 bar bei einer Temperatur im Bereich von 60 bis 220°C sieden, unter Erhalt von Ammoniak als Kopfprodukt und eines Sumpfes I, wobei man den Ammoniak nicht vollständig abtrennt.

Als Verbindung A kommen Substanzen in Betracht, die unter den Destillationsbedingungen inert sind und einen Siedepunkt im Bereich von 60 bis 250, vorzugsweise von 60 bis 150°C bei einem Druck von 18 bar aufweisen. Beispielhaft seien genannt: Alkane, Cycloalkane, Aromaten, Naphthene, Alkohole, Ether, Nitrile und Amine mit den zuvor genannten Eigenschaften, insbesondere C₅-C₈-Alkane und C₂-C₄-Alkanole, besonders bevorzugt n-Pentan, Cyclohexan, Triethylamin, Ethanol, Acetonitril, n-Hexan, Di-n-propylether, Isopropanol, n-Butylamin, Benzol, ganz besonders bevorzugt Ethanol.

Üblicherweise gibt man Verbindung A in einer Menge im Bereich von 0,1 bis 50, vorzugsweise von 1 bis 10 Gew.-%, bezogen auf den Sumpf I, zu.

Man unterwirft den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber dem Reaktoraustrag aus R1 einer zweiten Destillation unter Erhalt einer Mischung aus der(n) inerten Verbindung(en) A und Ammoniak als Kopfprodukt und eines Sumpfes II, wobei man die Destillation bei einer Sumpftemperatur im Bereich von 100 bis 250, vorzugsweise von 140 bis 200°C, und einem Druck im Bereich von 2 bis 15, vorzugsweise von 4 bis 12 bar durchführt, mit der Maßgabe, daß man die Drücke der ersten und der zweiten Kolonne (K2 in der Zeichnung) so aufeinander abstimmt, daß man bei einer jeweiligen Sumpftemperatur von maximal 250°C eine Kopftemperatur von über 20°C erhält. Es kann auch vorteilhaft sein, daß man die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
den Kopfabzug der zweiten Kolonne dampfförmig nach Druckerhöhung mit einem Verdichter in die erste Kolonne oder in deren Kondensator zurückführt.

Man unterwirft den Sumpf II, enthaltend im wesentlichen 6-Aminocapronitril, Hexamethylendiamin, Adipodinitril, Hexamethylenimin und inerte Verbindung(en) A in einer dritten Kolonne (K3 in der Zeichnung) einer Destillation unter Erhalt der inerten Verbindung(en) A als Kopfprodukt und eines Sumpfes III, wobei man die Destillation bei einer Sumpftemperatur im Bereich von 50 bis 250, vorzugsweise von 140 bis 200°C und einem Druck im Bereich von 0,05 bis 2, vorzugsweise von 0,2 bis 1 bar durchführt, mit der Maßgabe, daß man die als Kopfprodukt erhaltene(n) inerte(n) Verbindung(en) A der zweiten Kolonne zuführt, und gewünschtenfalls die Destillation in Gegenwart von einer oder mehreren unter den Destillationsbedingungen inerten Verbindung(en) B durchführt, die bei einem gegebenen Druck von 0,3 bar im Bereich von 20 bis 250, vorzugsweise von 60 bis 170°C sieden.

Als Verbindung B seien beispielhaft genannt:

Alkane, Cycloalkane, Aromaten, Naphthene, Alkohole, Ether, Nitrile und Amine mit den zuvor genannten Eigenschaften, insbesondere Di-n-butylether, Valeronitril, n-Octan, Cyclooctan, n-Hexylamin, Hexamethylenimin, Hexamethylendiamin bevorzugt Hexamethylenimin und/oder Hexamethylendiamin, besonders bevorzugt Hexamethylenimin.

In einer bevorzugten Ausführungsform wählt man als Verbindung B Hexamethylenimin und/oder Hexamethylendiamin, oder, besonders bevorzugt, man gibt keine weitere Verbindung B zu.

Bevorzugt führt man Verbindung B der Kolonne K3 in einer Menge im Bereich von 0,01 bis 50, vorzugsweise von 0,5 bis 10 Gew.-%, bezogen auf den Sumpf II, zu.

Man unterwirft den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne (K4 in der Zeichnung) einer Destillation unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und der Druck im Bereich von 0,05 bis 1,5 bar liegen unter Erhalt eines Sumpfes IV.

Gewünschtenfalls stattet man die Kolonne mit einer Trennwand im Bereich zwischen Zulauf und Seitenabzug aus (Petlyuk-Kolonne), so daß das gewonnene Hexamethylendiamin im wesentlichen frei ist von Hexamethylenimin und inerten Verbindung(en) B sowie von anderen Leichtsiedern, wobei

man Kopfprodukt KP1 und/oder HMD aus dem Seitenabzug SA1 bei Bedarf der dritten Kolonne zuführt oder gegebenenfalls nur teilweise der dritten Kolonne zuführt und den Rest ausschleust.

Man unterwirft den Sumpf IV, enthaltend im wesentlichen 6-Aminocapronitril und Adiponitril sowie eventuelle Hochsieder in einer fünften Kolonne (K5 in der Zeichnung) einer Destillation, unter Erhalt von 6-Aminocapronitril mit einer Reinheit von mindestens 95 %, vorzugsweise 99 bis 99,9 %, als Kopfprodukt und eines Seitenabzuges V, bestehend im wesentlichen aus Adipodinitril, sowie eines Sumpfes V, der aus Hochsiedern und geringen Mengen Adipodinitril besteht.

Gewünschtenfalls stattet man die Kolonne mit einer Trennwand im Bereich zwischen Zulauf und Seitenabzug aus, so daß das gewonnene Adipodinitril niedrigere Anteile an Hochsiedern enthält, wobei die Destillation bei einer Sumpftemperatur im Bereich von 50 bis 250°C und einem Druck im Bereich von 10 bis 300 mbar durchgeführt wird.

Anstatt das Adiponitril als Seitenabzug V zu erhalten, kann man auch den Sumpf V der Kolonne K5, enthaltend Adipodinitril und höhersiedenden Verbindungen, in einer weiteren Kolonne K6 destillativ auftrennen und dabei Adipodinitril als Kopfprodukt VI erhalten.

Erfindungsgemäß behandelt man den im wesentlichen Adipodinitril enthaltenden Teil, der bei der beschriebenen destillativen Aufarbeitung des bei der Hydrierung von Adipodinitril anfallenden Reaktionsgemisches als Seitenabzug V der Kolonne K5, als Kopfprodukt VI der Kolonne K6 oder als Sumpfprodukt der Kolonne D5, vorzugsweise als Seitenabzug V der Kolonne D5 anfällt, eine Säure oder einen sauren Ionentauscher zu.

Als Säuren oder saure Ionentauscher kommen in erster Linie Substanzen in Betracht, die gegenüber primären, sekundären und tertiären gesättigten und ungesättigten Aminen wie Enaminen als Protonendonatoren fungieren können. Besonders geeignet sind dazu Säuren mit einem pKₐ-Wert von höchstens 10, vorzugsweise höchstens 7.

Als Säuren können anorganische Säuren wie Salpetersäure, vorzugsweise Schwefelsäure insbesondere als 100 Gew.-%ige Schwefelsäure oder als eine mindestens 90 Gew.-%, vorzugsweise 96 Gew.-%, enthaltende Mischung insbesondere mit Wasser oder Phosphorsäure, organische Säuren, beispielsweise Carbonsäuren wie Adipinsäure, 2-Ethylhexansäure, Pimelinsäure, Korksäure, Undecandisäure, Terephthalsäure, Cyclohexancarbonsäure, beispielsweise Sulfonsäure wie p-Toluolsulfonsäure, Benzolsulfonsäure, als saure Ionentauscher beispielsweise Lewatit S100G1, Amberlyst 15, Dowex 50 WX 8, Bay. Kat. K 2431, Amberlite IR-120 sowie Gemische solcher Säuren und sauren Ionentauscher eingesetzt werden.

Die Umsetzung des Adiponitril mit der Säure kann in Gegenwart eines flüssigen Verdünnungsmittels wie Wasser erfolgen, wobei das flüssige Verdünnungsmittel zusammen mit der Säure oder vor oder nach der Säurezugabe zu dem Adiponitril gegeben werden kann.

Die direkte Behandlung des von höhersiedenden Verbindungen nicht befreiten Adiponitrils, beispielsweise das Sumpfprodukt V der Kolonne K5, wenn diese keinen Adipodinitril-Seitenabzug enthält, ist ebenso möglich. In diesem Fall erhöht sich der Verbrauch an Säure oder sauren Ionentauscher und die Menge des anfallenden Rückstandes nach der Abtrennung des Adipodinitril.

Das Molverhältnis der Säuregruppen zu den im Rückstand vorhandenen basischen Verbindungen sollte mindestens äquimolar, vorzugsweise überäquimolar sein. Als vorteilhaft haben sich Säurezugaben von 0,01 bis 10 Gew.-%, insbesondere 0,1 bis 2 Gew.-% bezogen auf Adipodinitril, erwiesen.

Die Umsetzung des Adiponitrils mit der Säure kann in an sich bekannter Weise, wie durch Mischen oder durch Leiten des Adiponitrils über ein Ionentauscher-Festbett, vorteilhaft bei Temperaturen von 2 bis 250°C, insbesondere 30 bis 100°C erfolgen, wodurch sich Reaktionszeiten von 1 Sekunde bis 30 Minuten, insbesondere 1 Sekunde bis 10 Minuten ergeben.

Aus der Mischung kann das Adipodinitril in an sich bekannter Weise, vorteilhaft destillativ oder extraktiv, abgetrennt werden.

Bei Zugabe eines flüssigen Verdünnungsmittel wie Wasser bei der Umsetzung des Rückstandes mit der Säure kann man das flüssige Verdünnungsmittel vor der Abtrennung des Adipodinitrils vorzugsweise adsorptiv, insbesondere destillativ abtrennen.

Ebenso können die nach der Säurezugabe erhaltenen Umsetzungsprodukte und gegebenenfalls überschüssige Säure vorteilhaft durch Extraktion, beispielsweise mit Wasser, von Adipodinitril abgetrennt werden.

Das nach dem erfindungsgemäßen Verfahren erhaltene Adipodinitril kann erneut für die partielle Hydrierung zu Hexamethylendiamin und 6-Aminocapronitril verwendet werden, wobei eine Aufpegelung von Nebenprodukten vermieden wird, die eine spezifikationsgerechte Herstellung von Hexamethylendiamin und 6-Aminocapronitril verhindern.

Das 6-Aminocapronitril kann anschließend in an sich bekannter Weise gegebenenfalls über die Zwischenstufe Caprolactam zu Polyamid 6, Hexamethylendiamin mit Adipinsäure zu Polymid 66 verarbeitet werden. Polyamid 6 und Polyamid 66 stellen technisch bedeutende Werkstoffe dar.

### Beispiel 1

a) Katalysatorherstellung
   Durch Tempern eines Gemischs aus Magnetit, Kaliumcarbonat, Al₂O₃, Calciumcarbonat, Brechen der erstarrten Schmelze und Sieben gemäß A.B. Stiles, T.A. Koch, Catalyst Manufacture (1995) S. 167/68 wurde eine oxidische Masse folgender Zusammensetzung erhalten: 1,1 Gew.-% K₂O, 3,0 Gew.-% Al₂O₃, 2,3 Gew.-% CaO, 0,11 Gew.-% Si, 0,01 Gew.-% Ti, Rest Fe-Oxide.
   Anschließend wurde diese Masse im N₂/H₂-Strom bei 450°C 72 h reduziert, bei Raumtemperatur mit einem N₂/Luft-Gemisch (24 h mit 1 % Luft in Stickstoff), wobei die Temperatur im Katalysatorbett nicht über 45°C stieg, passiviert und mit Wasser 7 Tage gewaschen.
   Die erhaltene Katalysatormasse hatte folgende Zusammensetzung: 1,2 Gew.-% Al, 0,74 Gew.-% Ca, 0,02 Gew.-% K, 0,11 Gew.-% Si, 0,01 Gew.-% Ti, Rest Fe/Fe-Oxid. Die Summe der Promotoren aus Gruppe b) liegt bei 1,32 Gew.-%, die Summe der Promotoren aus Gruppe c) gerechnet als Oxide, liegt bei 1,06 Gew.-%.
b) Partielle Hydrierung von ADN zu ACN
   Ein Rohrreaktor (Länge 180 cm, d = 30 mm) wurde mit 740 ml (1819 g) der nach (a) hergestellten Katalysatormasse befüllt und drucklos im Wasserstoffstrom (500 Nl/h) reduziert. Dabei wurde die Temperatur innerhalb von 24 h von 30°C auf 340°C angehoben und anschließend 72 h bei 340°C gehalten.
   Nach Absenken der Temperatur wurde dem Reaktor bei 250 bar ein Gemisch aus 400 ml/h ADN, welches aus Butadien und Blausäure hergestellt wurde und dessen P-Gehalt durch Chemisorption mit Calciumoxid auf <1 ppm abgesenkt wurde, 660 ml NH₃ und 500 Nl/h H₂ zugeführt.
   Nach einer Laufzeit von 1000 h bei einer Reaktionstemperatur von 120°C lag bei über die gesamte Laufzeit konstanten Umsatz von 70 % und konstanter Gesamtselektivität (ACN + HMD) von 99 %, die ACN-Selektivität konstant um 40 %.

### Vergleichs-Beispiel

a) Katalysatorherstellung
   Durch Tempern eines Gemischs aus Magnetit, Kaliumcarbonat, Al₂O₃, Calciumcarbonat, Brechen der erstarrten Schmelze und Sieben gemäß A.B. Stiles, T.A. Koch, Catalyst Manufacture (1995) S. 167/68 wurde eine oxidische Masse folgender Zusammensetzung erhalten: 1,1 Gew.-% K₂O, 3,0 Gew.-% Al₂O₃, 2,3 Gew.-% CaO, 0,11 Gew.-% Si, 0,01 Gew.-% Ti, Rest Fe-Oxide.
   Anschließend wurde diese Masse im N₂/H₂-Strom bei 450°C 72 h reduziert, bei Raumtemperatur mit einem N₂/Luft-Gemisch (24 h mit 1 % Luft in Stickstoff), wobei die Temperatur im Katalysatorbett nicht über 45°C stieg, passiviert und mit Wasser 7 Tage gewaschen.
   Die erhaltene Katalysatormasse hatte folgende Zusammensetzung: 1,2 Gew.-% Al, 0,74 Gew.-% Ca, 0,02 Gew.-% K, 0,11 Gew.-% Si, 0,01 Gew.-% Ti, Rest Fe/Fe-Oxid. Die Summe der Promotoren aus Gruppe b) liegt bei 1,32 Gew.-%, die Summe der Promotoren aus Gruppe c) gerechnet als Oxide, liegt bei 1,06 Gew.-%.
b) Partielle Hydrierung von ADN zu ACN
   Ein Rohrreaktor (Länge 180 cm, d = 30 mm) wurde mit 740 ml (1819 g) der nach (a) hergestellten Katalysatormasse befüllt und drucklos im Wasserstoffstrom (500 Nl/h) reduziert. Dabei wurde die Temperatur innerhalb von 24 h von 30°C auf 340°C angehoben und anschließend 72 h bei 340°C gehalten.
   Nach Absenken der Temperatur wurde dem Reaktor bei 250 bar ein Gemisch aus 400 ml/h ADN, welches aus Butadien und Blausäure hergestellt wurde und einen P-Gehalt von 4 ppm hatte, 660 ml NH3 und 500 Nl/h H2 zugeführt.
   Nach einer Laufzeit von 1000 h bei einer Reaktionstemperatur von 120°C war bei über die gesamte Laufzeit konstanten Umsatz von 70 % und konstanter Gesamtselektivität (ACN + HMD) von 99 %, die ACN-Selektivität von 40 % auf 30 % abgefallen.

## Patentansprüche

1. Verfahren zur gleichzeitigen Herstellung von 6-Aminocapronitril und Hexamethylendiamin ausgehend von einem Gemisch, das eine phosphorhaltige Verbindung und im wesentlichen Adipodinitril enthält und das durch Umsetzung von Butadien mit Cyanwasserstoffsäure in Gegenwart eines eine phosphorhaltige Verbindung enthaltenden Katalysators erhalten wurde, dadurch gekennzeichnet, daß man
(1) den Gewichtsanteil an einer phosphorhaltigen Verbindung, gerechnet als Phosphor, in dem Gemisch reduziert und
(2) die erhaltene Mischung in Gegenwart eines Katalysators zu einem alpha, omega-Aminonitril partiell hydriert und
(3) 6-Aminocapronitril und Hexamethylendiamin aus der Mischung abtrennt.

2. Verfahren nach Anspruch 1, dadurch gekennzeichnet, daß der Gewichtsanteil an einer phosphorhaltigen Verbindung gerechnet als Phosphor in der Mischung in Teilschritt (2) kleiner als 5 ppm bezogen auf Adipodinitril ist.

3. Verfahren nach den Ansprüchen 1 und 2, dadurch gekennzeichnet, daß der Gewichtsanteil an einer phosphorhaltigen Verbindung, gerechnet als Phosphor, in der Mischung in Teilschritt (2) kleiner als 1 ppm bezogen auf Adipodinitril beträgt.

4. Verfahren nach den Ansprüchen 1 bis 3, dadurch gekennzeichnet, daß man den Gewichtsanteil einer phosphorhaltigen Verbindung in Schritt (1) destillativ reduziert.

5. Verfahren nach den Ansprüchen 1 bis 3, dadurch gekennzeichnet, daß man den Gewichtsanteil einer phosphorhaltigen Verbindung in Schritt (1) extraktiv reduziert.

6. Verfahren nach den Ansprüchen 1 bis 3, dadurch gekennzeichnet, daß man den Gewichtsanteil einer phosphorhaltigen Verbindung in Schritt (1) durch Behandlung mit einer Base reduziert.

7. Verfahren nach den Ansprüchen 1 bis 3, dadurch gekennzeichnet, daß man den Gewichtsanteil einer phosphorhaltigen Verbindung in Schritt (1) adsorptiv oder chemisorptiv reduziert.

8. Verfahren nach den Ansprüchen 1 bis 7, wobei man die Hydrierung in Gegenwart eines flüssigen Verdünnungsmittels durchführt.

9. Verfahren nach den Ansprüchen 1 bis 8, wobei man das Verfahren in Gegenwart eines flüssigen Verdünnungsmittels durchführt und das flüssige Verdünnungsmittel im wesentlichen zwischen den Teilschritten (2) und (3) abtrennt.

10. Verfahren nach den Ansprüchen 1 bis 9, wobei man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a) 1 eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a) einer Verbindung auf der Basis eines Alkalimetalles oder eines Erdalkalimetalles, enthält, mit der Maßgabe, daß, wenn als Komponente (a) eine Verbindung auf der Basis von nur Ruthenium oder Rhodium oder Ruthenium und Rhodium oder Nickel und Rhodium gewählt wird, der Promotor (b) gewünschtenfalls entfallen kann sowie mit der weiteren Maßgabe, daß, die Komponente (a) nicht auf der Basis von Eisen besteht, wenn die Komponente (b) Aluminium ist.

11. Verfahren nach den Ansprüchen 1 bis 10, dadurch gekennzeichnet, daß man einen Katalysator verwendet, der
(a) Eisen oder eine Verbindung auf der Basis von Eisen enthält und
(b) von 0,01 bis 5 Gew.-% bezogen auf (a) eines Promotors auf der Basis eines Elementes oder 2, 3, 4 oder 5 Elementen ausgewählt aus der Gruppe bestehend aus Aluminium, Silizium, Titan, Zirkonium und Vanadium sowie
(c) von 0 bis 0,5 Gew.-% bezogen auf (a) einer Verbindung auf der Basis eines Alkali- oder Erdalkalimetalles, enthält.

12. Verfahren nach den Ansprüchen 1 bis 11, dadurch gekennzeichnet, daß man aus dem Hydriergemisch einen im wesentlichen Adipodinitril enthaltenden Teil abtrennt.

13. Verfahren nach Anspruch 12, dadurch gekennzeichnet, daß man dem im wesentlichen Adipodinitril enthaltenden Teil 0,01 bis 10 Gew.-% einer Säure, bezogen auf Adipodinitril, zusetzt und das Adipodinitril von dem mit Säure behandelten Gemisch abtrennt.

14. Verfahren nach den Ansprüchen 1 bis 13, dadurch gekennzeichnet, daß man das Adipodinitril von dem Hydriergemisch destillativ abtrennt und eine Säure einsetzt, deren Siedepunkt unter dem für die Destillation gewählten Druck über dem Siedepunkt von Adipodinitril liegt.

15. Verfahren nach den Ansprüchen 9 bis 14, dadurch gekennzeichnet, daß man eine Säure mit einem pKₐ-Wert von höchstens 10 einsetzt.

16. Verfahren nach den Ansprüchen 1 bis 15, dadurch gekennzeichnet, daß man das nach den Verfahren erhaltene Adipodinitril erneut zur gleichzeitigen Herstellung von 6-Aminocapronitril und Hexamethylendiamin ausgehend von Adipodinitril einsetzt.

## Claims

1. A process for coproduction of 6-aminocapronitrile and hexamethylenediamine starting from a mixture comprising a phosphorus-containing compound and essentially adiponitrile and obtained by reaction of butadiene with hydrocyanic acid in the presence of a catalyst comprising a phosphorus-containing compound, which comprises
(1) reducing the weight proportion of phosphorus-containing compound, calculated as phosphorus, in the mixture,
(2) partially hydrogenating the resulting mixture in the presence of a catalyst to form an α,ω―aminonitrile, and
(3) removing 6-aminocapronitrile and hexamethylenediamine from the mixture.

2. A process as claimed in claim 1, wherein the weight proportion of phosphorus-containing compound, calculated as phosphorus, in the mixture of step (2) is less than 5 ppm, based on adiponitrile.

3. A process as claimed in claim 1 or 2, wherein the weight proportion of phosphorus-containing compound, calculated as phosphorus, in the mixture of step (2) is less than 1 ppm, based on adiponitrile.

4. A process as claimed in any of claims 1 to 3, wherein the reducing of the weight proportion of phosphorus-containing compound in step (1) is effected by distillation.

5. A process as claimed in any of claims 1 to 3, wherein the reducing of the weight proportion of phosphorus-containing compound in step (1) is effected by extraction.

6. A process as claimed in any of claims 1 to 3, wherein the reducing of the weight proportion of phosphorus-containing compound in step (1) is effected by treatment with a base.

7. A process as claimed in any of claims 1 to 3, wherein the reducing of the weight proportion of phosphorus-containing compound in step (1) is effected by adsorption or chemisorption.

8. A process as claimed in any of claims 1 to 7, wherein the hydrogenation is carried out in the presence of a liquid diluent.

9. A process as claimed in any of claims 1 to 8 in the presence of a liquid diluent, further comprising removing the liquid diluent essentially between steps (2) and (3).

10. A process as claimed in any of claims 1 to 9, wherein the catalyst used comprises
(a) a compound based on a metal selected from the group consisting of nickel, cobalt, iron, ruthenium and rhodium,
(b) from 0.01 to 25, preferably from 0.1 to 5, % by weight, based on (a), of a promoter based on a metal selected from the group consisting of palladium, platinum, iridium, osmium, copper, silver, gold, chromium, molybdenum, tungsten, manganese, rhenium, zinc, cadmium, lead, aluminum, tin, phosphorus, arsenic, antimony, bismuth and rare earth metals, and
(c) from 0 to 5, preferably from 0.1 to 3, % by weight, based on (a), of a compound based on an alkali metal or an alkaline earth metal,
with the proviso that, if a compound based on only ruthenium or rhodium or ruthenium and rhodium or nickel and rhodium is chosen as component (a), said promoter (b) can, if desired, be dispensed with, and with the further proviso that said component (a) shall not be based on iron when said component (b) is aluminum.

11. A process as claimed in any of claims 1 to 10, wherein the catalyst used comprises
(a) iron or a compound based on iron,
(b) from 0.01 to 5% by weight, based on (a), of a promoter based on an element or 2, 3, 4 or 5 elements selected from the group consisting of aluminum, silicon, titanium, zirconium and vanadium, and
(c) from 0 to 0.5% by weight, based on (a), of a compound based on an alkali or alkaline earth metal.

12. A process as claimed in any of claims 1 to 11, further comprising removing from the hydrogenation mixture a portion comprising essentially adiponitrile.

13. A process as claimed in claim 12, further comprising admixing the portion comprising essentially adiponitrile with from 0.01 to 10% by weight of an acid, based on adiponitrile, and removing the adiponitrile from the acid-treated mixture.

14. A process as claimed in any of claims 1 to 13, wherein the adiponitrile is removed from the hydrogenation mixture by distillation and the acid used has a higher boiling point than adiponitrile under the distillation pressure.

15. A process as claimed in any of claims 9 to 14, wherein the acid used has a pKₐ value of not more than 10.

16. A process as claimed in any of claims 1 to 15, further comprising re-using the adiponitrile recovered by the processes for coproduction of 6-aminocapronitrile and hexamethylenediamine on the basis of adiponitrile.

## Revendications

1. Procédé de préparation simultanée de 6-amino-capronitrile et d'hexaméthylènediamine à partir d'un mélange contenant un composé phosphoré et essentiellement de l'adiponitrile et qui est obtenu par réaction de butadiène et d'acide cyanhydrique en présence d'un catalyseur contenant un composé phosphoré, caractérisé en ce que:
(1) la fraction pondérale d'un composé phosphoré, calculée en tant que phosphore, est réduite dans le mélange et
(2) le mélange obtenu est partiellement hydrogéné en un alpha, oméga-aminonitrile, en présence d'un catalyseur, et
(3) le 6-aminocapronitrile et l'hexaméthylènediamine sont séparés du mélange.

2. Procédé selon la revendication 1, caractérisé en ce que la fraction pondérale d'un composé phosphoré, calculée en tant que phosphore dans le mélange est, dans l'étape partielle (2), inférieure à 5 ppm par rapport à l'adiponitrile.

3. Procédé selon les revendications 1 et 2, caractérisé en ce que la fraction pondérale d'un composé phosphoré, calculée en tant que phosphore dans le mélange est, dans l'étape partielle (2), inférieure à 1 ppm par rapport à l'adiponitrile.

4. Procédé selon les revendications 1 à 3, caractérisé en ce que la fraction pondérale d'un composé phosphoré est réduite par distillation dans l'étape (1).

5. Procédé selon les revendications 1 à 3, caractérisé en ce que la fraction pondérale d'un composé phosphoré est réduite par extraction dans l'étape (1).

6. Procédé selon les revendications 1 à 3, caractérisé en ce que la fraction pondérale d'un composé phosphoré est réduite dans l'étape (1) par traitement au moyen d'une base.

7. Procédé selon les revendications 1 à 3, caractérisé en ce que la fraction pondérale d'un composé phosphoré est réduite dans l'étape (1) par adsorption ou chimisorption.

8. Procédé selon les revendications 1 à 7, caractérisé en ce que l'on entreprend l'hydrogénation en présence d'un diluant liquide.

9. Procédé selon les revendications 1 à 8, caractérisé en ce que l'on exécute le procédé en présence d'un diluant liquide et que l'on sépare essentiellement le diluant liquide entre les étapes partielles (2) et (3).

10. Procédé selon les revendications 1 à 9, dans lequel on utilise un catalyseur:
(a) contenant un composé à base d'un métal choisi dans le groupe formé par le nickel, le cobalt, le fer, le ruthénium et le rhodium, et
(b) contenant de 0,01 à 25, de préférence de 0,1 à 5% en poids, par rapport à (a), d'un promoteur à base d'un métal choisi dans le groupe formé par le palladium, le platine, l'iridium, l'osmium, le cuivre, l'argent, l'or, le chrome, le molybdène, le tungstène, le manganèse, le rhénium, le zinc, le cadmium, le plomb, l'aluminium, l'étain, le phosphore, l'arsenic, l'antimoine, le bismuth et les terres rares, ainsi que
(c) de 0 à 5, de préférence de 0,1 à 3% en poids, par rapport à (a), d'un composé à base d'un métal alcalin ou d'un métal alcalino-terreux,
avec la condition que, lorsque l'on choisit en tant que composant (a) un composé à base uniquement de ruthénium ou de rhodium, ou de ruthénium et de rhodium, ou de nickel et de rhodium, le promoteur (b) puisse éventuellement être abandonné, et avec la condition supplémentaire que le composant (a) ne soit pas à base de fer lorsque le composant (b) est de l'aluminium.

11. Procédé selon les revendications 1 à 10, caractérisé en ce que l'on utilise un catalyseur:
(a) contenant du fer ou un composé à base de fer et
(b) de 0,01 à 5% en poids, par rapport à (a), d'un promoteur à base d'un élément ou de 2, 3, 4 ou 5 éléments choisis dans le groupe formé par l'aluminium, le silicium, le titane, le zirconium et le vanadium, ainsi que
(c) de 0 à 0,5% en poids, par rapport à (a), d'un composé à base d'un métal alcalin ou alcalino-terreux.

12. Procédé selon les revendications 1 à 11, caractérisé en ce que l'on sépare du mélange d'hydrogénation une fraction contenant essentiellement de l'adiponitrile.

13. Procédé selon la revendication 12, caractérisé en ce que l'on ajoute, à la fraction contenant essentiellement de l'adiponitrile, de 0,01 à 10% en poids d'un acide, par rapport à l'adiponitrile, et que l'on sépare l'adiponitrile du mélange traité par l'acide.

14. Procédé selon les revendications 1 à 13, caractérisé en ce que l'on sépare par distillation l'adiponitrile du mélange d'hydrogénation et que l'on met en oeuvre un acide dont le point d'ébullition est supérieur au point d'ébullition de l'adiponitrile à la pression choisie pour la distillation.

15. Procédé selon les revendications 9 à 14, caractérisé en ce que l'on met en oeuvre un acide présentant un pKₐ d'un maximum de 10.

16. Procédé selon les revendications 1 à 15, caractérisé en ce que l'on remet en oeuvre l'adiponitrile obtenu après le procédé pour la production simultanée de 6-aminocapronitrile et d'hexaméthylènediamine à partir d'adiponitrile.
